# EUROPEAN PATENT APPLICATION

(11) **EP 3 514 171 A1**
(43) Date of publication of application: **24.07.2019**
(21) Application number: 19152679.7
(22) Date of filing: 18.01.2019
(51) Int. Cl.: C07K 14/61, A61K 38/27, C12N 15/09

(54) **LONG-ACTING THERAPEUTIC FUSION PROTEINS**

(30) Priority: 18.01.2018 US 201862619091 P
(71) Applicant: Molecular Cloning Laboratories (MCLAB) LLC, South San Francisco, CA 94080 (US)
(72) Inventor: YAN, Zhe, South San Francisco, CA 94080 (US); SHI, Changping, South San Francisco, CA 94080 (US); SHEN, Dan, South San Francisco, CA 94080 (US)
(74) Representative: FRKelly

(57) **Abstract**

Chimeric Fc fusion polypeptides are provided, optionally including biologically active polypeptides for therapeutic use.

SEQUENCE LISTING

<110> MOLECULAR CLONING LABORATORIES (MCLAB) LLC

<120> LONG-ACTING THERAPEUTIC FUSION PROTEINS

<130> 05185.002EP1

<140>
<141>

<150> 62/619,091
<151> 2018-01-18

<160> 6

<170> PatentIn version 3.5

<210> 1
<211> 10
<212> PRT
<213> Homo sapiens

<400> 1
Asp Lys Thr His Thr Cys Pro Pro Cys Pro
1               5                   10

<210> 2
<211> 10
<212> PRT
<213> Artificial Sequence

<220>
<221> source
<223> /note="Description of Artificial Sequence: Synthetic
      peptide"

<400> 2
Asp Lys Thr His Thr Ser Pro Pro Cys Pro
1               5                   10

<210> 3
<211> 10
<212> PRT
<213> Artificial Sequence

<220>
<221> source
<223> /note="Description of Artificial Sequence: Synthetic
      peptide"

<400> 3
Asp Lys Thr His Thr Cys Pro Pro Ser Pro
1               5                   10

<210> 4
<211> 217
<212> PRT
<213> Homo sapiens

<400> 4
Ala Pro Glu Phe Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser Gln Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Gly Leu Pro Ser Ser Ile Glu Lys Thr Ile Ser Lys Ala Lys Gly Gln
            100                 105                 110

Pro Arg Glu Pro Gln Val Tyr Thr Leu Pro Pro Ser Gln Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Arg Leu Thr Val Asp Lys Ser Arg Trp Gln Glu Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys
    210                 215

<210> 5
<211> 5
<212> PRT
<213> Artificial Sequence

<220>

<221> source
<223> /note="Description of Artificial Sequence: Synthetic
      peptide"

<400> 5
Gly Gly Gly Gly Ser
1               5


<210> 6
<211> 192
<212> PRT
<213> Homo sapiens

<400> 6
Met Phe Pro Thr Ile Pro Leu Ser Arg Leu Phe Asp Asn Ala Met Leu
1               5                   10                  15

Arg Ala His Arg Leu His Gln Leu Ala Phe Asp Thr Tyr Gln Glu Phe
            20                  25                  30

Glu Glu Ala Tyr Ile Pro Lys Glu Gln Lys Tyr Ser Phe Leu Gln Asn
        35                  40                  45

Pro Gln Thr Ser Leu Cys Phe Ser Glu Ser Ile Pro Thr Pro Ser Asn
    50                  55                  60

Arg Glu Glu Thr Gln Gln Lys Ser Asn Leu Glu Leu Leu Arg Ile Ser
65                  70                  75                  80

Leu Leu Leu Ile Gln Ser Trp Leu Glu Pro Val Gln Phe Leu Arg Ser
                85                  90                  95

Val Phe Ala Asn Ser Leu Val Tyr Gly Ala Ser Asp Ser Asn Val Tyr
            100                 105                 110

Asp Leu Leu Lys Asp Leu Glu Glu Gly Ile Gln Thr Leu Met Gly Arg
        115                 120                 125

Leu Glu Asp Gly Ser Pro Arg Thr Gly Gln Ile Phe Lys Gln Thr Tyr
    130                 135                 140

Ser Lys Phe Asp Thr Asn Ser His Asn Asp Asp Ala Leu Leu Lys Asn
145                 150                 155                 160

Tyr Gly Leu Leu Tyr Cys Phe Arg Lys Asp Met Asp Lys Val Glu Thr
                165                 170                 175

Phe Leu Arg Ile Val Gln Cys Arg Ser Val Glu Gly Ser Cys Gly Phe
            180                 185                 190

## Description

### FIELD OF THE INVENTION

The invention relates to chimeric polypeptides, particularly Fc fusion proteins for therapeutic applications.

### BACKGROUND

Therapeutic protein drugs are an important class of medicines serving patients most in need of novel therapies. More than 180 therapeutic proteins and peptides have been approved by the FDA to treat a wide variety of clinical indications, including cancers, autoimmunity/inflammation, exposure to infectious agents, and genetic disorders. However, many of these proteins and peptides have less than optimal pharmacokinetic properties, often because they are smaller than the kidney filtration cutoff of around 70 kDa and/or are subject to metabolic turnover by proteases, which significantly limits their *in vivo* half-life.

For example, growth hormone deficiency (GHD) during childhood results in growth retardation, as well as abnormal body composition, with more body fat than lean body mass, and decreased physical capacity and quality of life. Growth hormone (GH) treatment has been an established therapy for GHD in children and adults for more than three decades. GH treatment improves height, body composition, bone density, cardiovascular risk factors, physical fitness, and quality of life, and the treatment has few adverse side effects.

hGH has a plasma half-life of 3.4 hours after subcutaneous injection, and about 20 minutes after intravenous injection. Currently, daily subcutaneous injection to children with GHD is the standard treatment regimen. However, daily subcutaneous injections are inconvenient, painful, and distressing for many patients, resulting in noncompliance, reduced efficacy, and increased health care costs. There is therefore a strong need to develop long-acting hGH formulations that require less frequent administration, in order to improve patient compliance.

There are several approaches for increasing the stability and/or reducing renal filtration and elimination of therapeutic peptides and proteins, by increasing the size of the therapeutic polypeptide. One approach is a fusion with the Fc region of immunoglobulin G (IgG). Human IgG isotypes 1, 2, and 4 bind to neonatal Fc receptor (FcRn) in a pH-dependent manner to effect their recycling by epithelial cells. (Roopenian, DC, et al. (2007) Nat Rev Immunol 7(9):715-25) This binding occurs via specific residues in the Fc region of the antibody, giving these IgG isotypes a nominal 2- to 3-week half-life in human serum. (Strohl, WR, et al. (2012) Therapeutic antibody engineering: current and future advances driving the strongest growth area in the pharma industry. Cambridge: Woodhead Publishing Series in Biomedicine No. 11)

Taking advantage of size increase and the natural recycling process of IgG, Fc fusion proteins are thought to be protected from degradation by recycling. Many Fc fusion proteins of various types have been made over the past 35 years, virtually all of which were intended to prolong the half-life of a protein or peptide. As of May, 2015, eleven Fc fusion proteins had been approved for marketing by the FDA. Fc fusion proteins have been implemented for treatment of many disease conditions to provide longer dosing intervals, such as weekly or bi-weekly administration. Examples include etanercept (Enbrel) for treatment of various forms of arthritis, aflibercept (Eylea) for treatment of neovascular age-related macular degeneration, and dulaglutide (Trulicity) for treatment of type 2 diabetes. Overall, Fc fusion proteins have proven to be a successful alternative to improve pharmacological properties of therapeutic drugs with low immunogenic potential.

Fc fusion also results in increased expression and/or secretion, and Protein A affinity purification of Fc fusion proteins simplifies the downstream purification of the protein drug. As with antibodies, purification of the therapeutic compound may also leverage the Fc region, which binds reversibly and with high affinity to staphylococcal protein A or streptococcal protein G. Thus, fusion proteins containing an Fc fragment can easily be purified by affinity chromatography techniques using resin-conjugated protein A or protein G. (Ghose, et al. (2006) J Chromatogr A 1122:144-52)

Fc fusion based protein drug products that are currently on the market mostly include Fc from IgG1. The effector functions of the Fc region from IgG1 are exhibited, which results in undesirable properties. (U.S. Patent No. 5,49,053) Via effector functions of the Fc region, the fusion proteins can fix complement or bind to Fc receptor (FcR) expressing cells to produce antibody-dependent cell mediated cytotoxicity (ADCC) or complement-dependent cytotoxicity (CDC), causing side reactions, such as destruction of particular cells, and inducing production and secretion of various cytokines, thereby inducing inflammation. It would be desirable to develop a Fc fusion platform for therapeutic applications that does not produce these undesirable side effects.

One method to delete or reduce undesirable effector functions, while maintaining a high serum concentration of an immunoglobulin, is to use the IgG4 Fc to replace the IgG1 Fc region. In contrast to IgG1 and IgG2, IgG4 does not exhibit complement activation. In addition, IgG4 has lower Fc RI, Fc RII, and Fc Rllla/b receptor affinities than IgG1. Therefore, the Fc region of IgG4 is much less cytotoxic. IgG4 is considered to be an attractive therapeutic monoclonal antibody format when effector function is not desired. (Jiang, X-R, et al. (2011) Nat Rev Drug Discov 10:101-111) For example, pembrolizumab and nivolumab, anti-PD-1 (programmed death-1) IgG4 cancer therapeutics, both approved in the US in 2014, inhibit the interaction between the immunoinhibitory T-cell PD-1 receptor and its ligands but do not elicit ADCC or complement-dependent cytotoxicity. (Hamid, O., et al. (2013) N Engl J Med 369:134-144; Wang, C., et al. (2014) Cancer Immunol Res 2:846-856)

While the IgG4 core hinge contains equivalent cysteine residues, it also contains a Pro228Ser substitution in comparison to IgG1, which is suggested to promote a more flexible hinge region, resulting in formation of intra- rather than inter-heavy chain disulfide bonds. This imparts an intriguing property to IgG4, Fab-arm exchange (FAE) (Aalberse, R.C., et al. (2002) Immunology 105:9-19)

Fc fusion proteins have primarily been produced in mammalian expression systems. Such systems produce heterogeneous protein structures, due to glycosylation, and require expensive culture media, long production times, and costly viral inactivation steps. Depending on the strain of the host cell and the culture conditions, abnormal glycosylation patterns can result in antibodies that are less potent or even immunogenic. (Patel, et al. (1993) Biochem J 285:839-45; Walsh, G., et al. (2006) Nat Biotechnol 24:1241-52) CHO and other murine cell lines are known, for example, to add non-human, and thereby strongly immunogenic, sugar residues to therapeutic antibodies.

Attempts have been made to produce gylosylated antibodies via deletion of glycosylation pathways, resulting in the next generation of antibody therapeutics. Aglycosylated IgG behaves similarly to glycosylated IgG, in terms of antibody binding affinity, solubility, stability at physiological temperature (37 °C) and at 4 °C, and *in vivo* serum half-life (Hirstodorov, et al. (2013) Mol Biotechnol 53:326-335). Further, clinical trials with aglycosylated antibodies have reported no immunogenicity issues and no significant binding to FcRs or elicitation of ADCC or antibody-dependent-cellular-phagocytosis (ADCP). (Ju, M.S., et al. (2014) Curr Opin Biotechnol 30:128-39); Higel, F., et al. (2016) Eur J Pharm Biopharm 100:94-100)

Fc fusions with small peptides (peptibodies), such as Romiplostim (AMG531), have been produced in E. *coli.* This is a more rapid and economical method to scale up than mammalian culture systems. However, the fusion proteins are expressed as insoluble inclusion bodies, which require significant downstream processing steps, such as denaturation, refolding, and cysteine disulfide formation. Development of an Fc fusion platform that can be expressed in a bacterial system in a soluble form, rather than as inclusion bodies, would be desirable.

### BRIEF SUMMARY OF THE INVENTION

Chimeric polypeptides, polynucleotides encoding the chimeric polypeptides, and methods of making and using the chimeric polypeptides, are provided herein.

In one aspect, a chimeric polypeptide is provided, having the formula: H - Fc. wherein H is the hinge region of IgG1, and Fc is the Fc region of IgG4. In some embodiments, H and Fc are human polypeptide sequences. In one embodiment, H includes the amino acid sequence depicted in SEQ ID NO:1, or a sequence having at least about 80% sequence identity thereof. In other embodiments, H includes the amino acid sequence depicted in SEQ ID NO:2 or SEQ ID NO:3. In one embodiment, Fc includes the amino acid sequence depicted in SEQ ID NO:4, or a sequence having at least about 80% sequence identity thereof.

In some embodiments, a chimeric polypeptide is provided, having the formula:L-H-Fc, wherein L is a peptide linker. In some embodiments, L may include about 5 to about 20 amino acids. In one embodiment, L includes the amino acid sequence depicted in SEQ ID NO:5.

In some embodiments, a chimeric polypeptide is provided, having the formula: P-L-H-Fc, wherein P is a biologically active polypeptide. For example, the biologically active polypeptide may include a hormone, a cytokine, a growth factor, a co-stimulatory molecule, a hormone receptor, a cytokine receptor, a growth factor receptor, a short peptide, or an antibody fragment. In some embodiments, the biologically active polypeptide includes an antibody fragment, such as a Fab fragment, a V_{H} fragment, a V_{L} fragment, or a camelid nanobody. In one embodiment, the biologically active polypeptide is human growth hormone (hGH), for example, hGH having the amino acid sequence depicted in SEQ ID NO:6, or a sequence having at least about 80% sequence identity thereof. In other embodiments, the biologically active peptide is GLP-1, TNFR, Factor VIII, VEGFR, or TGF-beta1.

In some embodiments, a dimerized chimeric polypeptide is provided that includes a dimer of identical chimeric polypeptides as described herein, wherein the dimer includes at least one interchain disulfide bond in the hinge region. In some embodiments, the dimer includes one or two interchain disulfide bonds in the hinge region. In some embodiments, the dimerized chimeric polypeptide includes a dimer of identical chimeric polypeptides that include a biologically active polypeptide as described herein.

In another aspect, polynucleotides are provided that encode chimeric polypeptides as described herein. In some embodiments, the polynucleotide is provided in an expression vector.

In another aspect, host cells are provided that include an expression vector with a polynucleotide that encodes a chimeric polypeptide as described herein. In some embodiments, the host cell is a bacterial cell, for example, an E. *coli* cell.

In another aspect, methods are provided for producing chimeric polypeptides as described herein. The methods include culturing a host cell that includes a polynucleotide that encodes the chimeric polypeptide, for example, a host cell that includes an expression vector that includes a polynucleotide that encodes the chimeric polypeptide, under conditions suitable for expression of the chimeric polypeptide. In some embodiments, the host cell is a bacterial cell, for example, an E. *coli* cell. In some embodiments, the chimeric polypeptide is produced in a soluble form in the cytoplasm of the host cell, for example, as a soluble dimer.

In one embodiment, the host cell is an *E. coli* cell, cultured at a temperature of about 20°C to about 25°C, and the chimeric polypeptide is produced in a soluble form in the cytoplasm. In one embodiment, the culture conditions further include mild conditions for induction of beta-galactosidase activity, such as, for example, inclusion of a low concentration of an inducing reagent, e.g., isopropyl beta-D-1-thiogalactopyranoside (IPTG), or lactose, in the culture medium.

In some embodiments, the chimeric polypeptide is produced at a titer of about 1 g/liter to about 3 g/liter and a yield of about 30% to about 50%.

In some embodiments, the chimeric polypeptide is purified from the host cells by a method including extraction and/or chromatographic purification. For example, purification of the chimeric polypeptide may include one or more chromatographic purification steps, such as affinity chromatography, ion-exchange chromatography, hydrophobic chromatography, size-exclusion chromatography, or combinations thereof.

In another aspect, a pharmaceutical composition is provided that includes a chimeric polypeptide including a biologically active polypeptide as described herein, and a pharmaceutically acceptable carrier. In some embodiments, dosage forms are provided that include a therapeutically effective dose of a pharmaceutical composition as described herein.

In another aspect, methods are provided for treatment of a condition. The methods include administering a therapeutically effective amount of a pharmaceutical composition that includes a chimeric polypeptide including a biologically active polypeptide as described herein to an individual in need thereof, wherein the biologically active polypeptide treats the condition. In some embodiments, the individual is a mammal, for example, a human.

The biologically active polypeptide in the chimeric polypeptide may include a longer circulating half life in the individual than the biologically active polypeptide alone. In one embodiment, the circulating half life of the biologically active polypeptide in the individual is increased by about 6% to about 10%, in comparison to the circulating half life of the biologically active polypeptide alone.

In one embodiment, administration of the pharmaceutical condition is via subcutaneous injection.

In some embodiments, the condition that is treated includes hGH deficiency, and the biologically active polypeptide is hGH. In some embodiments, the therapeutically effective amount includes a weekly dosage of about 0.8 mg/kg to about 1.2 mg/kg or a twice monthly dosage of about 1.6 mg/kg to about 2.4 mg/kg.

In another aspect, kits are provided that include chimeric polypeptides, polynucleotides, host cells, and/or compositions as described herein. In one embodiment, the kit includes an expression vector for expression of a chimeric polypeptide as described herein. In one embodiment, the kit includes a host cell that includes an expression vector for production of a chimeric polypeptide as described herein.

### BRIEF DESCRIPTION OF THE DRAWINGS

**Figure 1** schematically depicts a chimeric polypeptide dimer as described herein.
**Figure 2** shows an SDS-PAGE analysis of hGH fusion polypeptide MC2-B in the supernatant and pellet of an *E*. *coli* cell lysate, as described in Example 2.
**Figure 3** shows an SDS-PAGE analysis of hGH fusion polypeptide purified from the supernatant of an *E. coli* cell lysate, as described in Example 3.
**Figure 4** shows binding affinity of hGH fusion polypeptide to hGH receptor, in comparison to recombinant hGH, as described in Example 4.
**Figure 5** shows the plasma level of hGH fusion polypeptide in rats over time, as described in Example 6.
**Figure 6** shows dose dependent increase in body weight in hypophysectomized rats, as described in Example 7.

### DETAILED DESCRIPTION

Chimeric Fc fusion polypeptides, and methods of making and using the chimeric polypeptides, are provided herein. The chimeric polypeptides herein include an IgG4 Fc region coupled to an IgG1 hinge region, and may be produced in soluble form, for example, in a bacterial system, such as in *Escherichia coli* (*E. coli*). The chimeric fusion polypeptide may include a biologically active polypeptide coupled to the IgG1 hinge region, optionally with a flexible peptide linker sequence between the biologically active polypeptide and the hinge sequence. Chimeric fusion polypeptides with biologically active polypeptides, as described herein, may be used for treatment of a condition in an individual, particularly in contexts in which a long half-life therapeutic would be desirable to provide long dosing intervals.

Unless defined otherwise herein, all technical and scientific terms used herein have the same meaning as commonly understood by one of ordinary skill in the art to which this invention belongs. Singleton, et al., Dictionary of Microbiology and Molecular Biology, second ed., John Wiley and Sons, New York (1994), and Hale & Markham, The Harper Collins Dictionary of Biology, Harper Perennial, NY (1991) provide one of skill with a general dictionary of many of the terms used in this invention. Any methods and materials similar or equivalent to those described herein can be used in the practice or testing of the present invention.

The practice of the present invention will employ, unless otherwise indicated, conventional techniques of molecular biology (including recombinant techniques), microbiology, cell biology, and biochemistry, which are within the skill of the art. Such techniques are explained fully in the literature, for example, Molecular Cloning: A Laboratory Manual, second edition (Sambrook et al., 1989); Oligonucleotide Synthesis (M. J. Gait, ed., 1984; Current Protocols in Molecular Biology (F. M. Ausubel et al., eds., 1994); PCR: The Polymerase Chain Reaction (Mullis et al., eds., 1994); and Gene Transfer and Expression: A Laboratory Manual (Kriegler, 1990).

Numeric ranges provided herein are inclusive of the numbers defining the range.

Unless otherwise indicated, nucleic acids are written left to right in 5' to 3' orientation; amino acid sequences are written left to right in amino to carboxy orientation, respectively.

### Definitions

"A," "an" and "the" include plural references unless the context clearly dictates otherwise.

As used herein, the term "polynucleotide" refers to a polymeric form of nucleotides of any length and any three-dimensional structure and single- or multi-stranded (e.g., single-stranded, doublestranded, triple-helical, etc.), which contain deoxyribonucleotides, ribonucleotides, and/or analogs or modified forms of deoxyribonucleotides or ribonucleotides, including modified nucleotides or bases or their analogs. Because the genetic code is degenerate, more than one codon may be used to encode a particular amino acid, and the present invention encompasses polynucleotides which encode a particular amino acid sequence. Any type of modified nucleotide or nucleotide analog may be used, so long as the polynucleotide retains the desired functionality under conditions of use, including modifications that increase nuclease resistance (e.g., deoxy, 2'-O-Me, phosphorothioates, etc.). Labels may also be incorporated for purposes of detection or capture, for example, radioactive or nonradioactive labels or anchors, e.g., biotin. The term polynucleotide also includes peptide nucleic acids (PNA). Polynucleotides may be naturally occurring or non-naturally occurring. The terms "polynucleotide," "nucleic acid," and "oligonucleotide" are used herein interchangeably. Polynucleotides may contain RNA, DNA, or both, and/or modified forms and/or analogs thereof. A sequence of nucleotides may be interrupted by non-nucleotide components. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR.sub.2 ("amidate"), P(O)R, P(O)OR', CO or CH.sub.2 ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. Polynucleotides may be linear or circular or comprise a combination of linear and circular portions.

As used herein, "polypeptide" refers to a composition comprised of amino acids and recognized as a protein by those of skill in the art. The conventional one-letter or three-letter code for amino acid residues is used herein. The terms "polypeptide" and "protein" or are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. A short polypeptide sequence may be termed a "peptide."

As used herein, a "vector" refers to a polynucleotide sequence designed to introduce nucleic acids into one or more cell types. Vectors include cloning vectors, expression vectors, shuttle vectors, plasmids, phage particles, cassettes and the like.

As used herein, the term "expression" refers to the process by which a polypeptide is produced based on the nucleic acid sequence of a gene. The process includes both transcription and translation.

As used herein, "expression vector" refers to a DNA construct containing a DNA coding sequence (*e.g.,* gene sequence) that is operably linked to one or more suitable control sequence(s) capable of effecting expression of the coding sequence in a host. Such control sequences include a promoter to effect transcription, an optional operator sequence to control such transcription, a sequence encoding suitable mRNA ribosome binding sites, and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. The plasmid is the most commonly used form of expression vector. However, the invention is intended to include such other forms of expression vectors that serve equivalent functions and which are, or become, known in the art.

A "promoter" refers to a regulatory sequence that is involved in binding RNA polymerase to initiate transcription of a gene. A promoter may be an inducible promoter or a constitutive promoter. An "inducible promoter" is a promoter that is active under environmental or developmental regulatory conditions.

The term "operably linked" refers to a juxtaposition or arrangement of specified elements that allows them to perform in concert to bring about an effect. For example, a promoter is operably linked to a coding sequence if it controls the transcription of the coding sequence.

"Under transcriptional control" is a term well understood in the art that indicates that transcription of a polynucleotide sequence depends on its being operably linked to an element which contributes to the initiation of, or promotes transcription.

"Under translational control" is a term well understood in the art that indicates a regulatory process which occurs after mRNA has been formed.

A "gene" refers to a DNA segment that is involved in producing a polypeptide and includes regions preceding and following the coding regions as well as intervening sequences (introns) between individual coding segments (exons).

As used herein, the term "host cell" refers to a cell or cell line into which a recombinant expression vector for production of a polypeptide may be transfected for expression of the polypeptide. Host cells include progeny of a single host cell, and the progeny may not necessarily be completely identical (in morphology or in total genomic DNA complement) to the original parent cell due to natural, accidental, or deliberate mutation. A host cell includes cells transfected or transformed *in vivo* with an expression vector.

The term "recombinant" refers to genetic material (*i.e.,* nucleic acids, the polypeptides they encode, and vectors and cells comprising such polynucleotides) that has been modified to alter its sequence or expression characteristics, such as by mutating the coding sequence to produce an altered polypeptide, fusing the coding sequence to that of another gene, placing a gene under the control of a different promoter, expressing a gene in a heterologous organism, expressing a gene at a decreased or elevated levels, expressing a gene conditionally or constitutively in manner different from its natural expression profile, and the like. Generally recombinant nucleic acids, polypeptides, and cells based thereon, have been manipulated by man such that they are not identical to related nucleic acids, polypeptides, and cells found in nature.

The term "selective marker" or "selectable marker" refers to a gene capable of expression in a host cell that allows for ease of selection of those hosts containing an introduced nucleic acid or vector. Examples of selectable markers include but are not limited to antimicrobial substances (e.g., hygromycin, bleomycin, or chloramphenicol) and/or genes that confer a metabolic advantage, such as a nutritional advantage, on the host cell.

The term "derived from" encompasses the terms "originated from," "obtained from," "obtainable from," "isolated from," and "created from," and generally indicates that one specified material finds its origin in another specified material or has features that can be described with reference to another specified material.

The term "culturing" refers to growing a population of cells, *e.g.,* microbial cells, under suitable conditions for growth, in a liquid or solid medium.

"Titer" refers to amount of a substance produced by a microorganism per unit volume in a

"Yield" refers to amount of a product produced from a feed material (for example, sugar) relative to the total amount that of the substance that would be produced if all of the feed substance were converted to product.

The term "heterologous," with reference to a polynucleotide or protein, refers to a polynucleotide or protein that does not naturally occur in a specified cell, *e.g.,* a host cell. It is intended that the term encompass proteins that are encoded by naturally occurring genes, mutated genes, and/or synthetic genes. In contrast, the term "homologous," with reference to a polynucleotide or protein, refers to a polynucleotide or protein that occurs naturally in the cell.

The term "introduced," in the context of inserting a nucleic acid sequence into a cell, includes "transfection," "transformation," or "transduction" and refers to the incorporation of a nucleic acid sequence into a eukaryotic or prokaryotic cell wherein the nucleic acid sequence may be incorporated into the genome of the cell (*e.g.,* chromosome, plasmid, plastid, or mitochondrial DNA), converted into an autonomous replicon, or transiently expressed.

"Transfection" or "transformation" refers to the insertion of an exogenous polynucleotide into a host cell. The exogenous polynucleotide may be maintained as a non-integrated vector, for example, a plasmid, or alternatively, may be integrated into the host cell genome. The term "transfecting" or "transfection" is intended to encompass all conventional techniques for introducing nucleic acid into host cells. Examples of transfection techniques include, but are not limited to, calcium phosphate precipitation, DEAE-dextran-mediated transfection, lipofection, electroporation, and microinjection.

As used herein, the terms "transformed," "stably transformed," and "transgenic" refer to a cell that has a non-native (*e.g.,* heterologous) nucleic acid sequence integrated into its genome or as an episomal plasmid that is maintained through multiple generations.

The terms "recovered," "isolated," "purified," and "separated" as used herein refer to a material (*e.g.,* a protein, nucleic acid, or cell) that is removed from at least one component with which it is naturally associated. For example, these terms may refer to a material which is substantially or essentially free from components which normally accompany it as found in its native state, such as, for example, an intact biological system.

Related (and derivative) proteins encompass "variant" proteins. Variant proteins differ from a parent protein and/or from one another by a small number of amino acid residues. In some embodiments, the number of different amino acid residues is any of about 1, 2, 3, 4, 5, 10, 20, 25, 30, 35, 40, 45, or 50. In some embodiments, variants differ by about 1 to about 10 amino acids. Alternatively or additionally, variants may have a specified degree of sequence identity with a reference protein or nucleic acid, e.g., as determined using a sequence alignment tool, such as BLAST, ALIGN, and CLUSTAL (see, infra). For example, variant proteins or nucleic acid may have at least about 35%, 40%, 45%, 50%, 55%, 60%, 65%, 70%, 75%, 80%, 85%, 86%, 87%, 88%, 89%, 90%, 91%, 92%, 93%, 94%, 95%, 96%, 97%, 98%, 99%, or even 99.5% amino acid sequence identity with a reference sequence.

As used herein, the term "analogous sequence" refers to a polypeptide sequence within a protein that provides a similar function, tertiary structure, and/or conserved residues with respect to a reference protein. For example, in epitope regions that contain an alpha helix or a beta sheet structure, replacement amino acid(s) in an analogous sequence maintain the same structural element. In some embodiments, analogous sequences are provided that result in a variant enzyme exhibiting a similar or improved function with respect to the parent protein from which the variant is derived.

As used herein, "homologous protein" refers to a protein that has similar function and/or structure as a reference protein . Homologs may be from evolutionarily related or unrelated species. In some embodiments, a homolog has a quaternary, tertiary and/or primary structure similar to that of a reference protein, thereby potentially allowing for replacement of a segment or fragment in the reference protein with an analogous segment or fragment from the homolog, with reduced disruptiveness of structure and/or function of the reference protein in comparison with replacement of the segment or fragment with a sequence from a non-homologous protein.

As used herein, "wild-type," "native," and "naturally-occurring" proteins are those found in nature. The terms "wild-type sequence" refers to an amino acid or nucleic acid sequence that is found in nature or naturally occurring. In some embodiments, a wild-type sequence is the starting point of a protein engineering project, for example, production of variant proteins.

The phrases "substantially similar" and "substantially identical" in the context of at least two nucleic acids or polypeptides typically means that a polynucleotide or polypeptide comprises a sequence that has at least about 35%, 40%, 45%, 50%, 55%, 60%, 65%, 70%, 75%, 80%, 85%, 86%, 87%, 88%, 89%, 90%, 91%, 92%, 93%, 94%, 95%, 96%, 97%, 98%, 99%, or even 99.5% sequence identity, in comparison with a reference (*e.g.,* wild-type) polynucleotide or polypeptide. Sequence identity may be determined using known programs such as BLAST, ALIGN, and CLUSTAL using standard parameters. (See, e.g., Altshul et al. (1990) J. Mol. Biol. 215:403-410; Henikoff et al. (1989) Proc. Natl. Acad. Sci. 89:10915; Karin et al. (1993) Proc. Natl. Acad. Sci. 90:5873; and Higgins et al. (1988) Gene 73:237). Software for performing BLAST analyses is publicly available through the National Center for Biotechnology Information. Also, databases may be searched using FASTA (Pearson et al. (1988) Proc. Natl. Acad. Sci. 85:2444-2448.) In some embodiments, substantially identical polypeptides differ only by one or more conservative amino acid substitutions. In some embodiments, substantially identical polypeptides are immunologically cross-reactive. In some embodiments, substantially identical nucleic acid molecules hybridize to each other under stringent conditions (*e.g.,* within a range of medium to high stringency).

An "antibody" is an immunoglobulin molecule capable of specific binding to a target, such as a carbohydrate, polynucleotide, lipid, polypeptide, etc., through at least one antigen recognition site, located in the variable region of the immunoglobulin molecule. As used herein, the term encompasses not only intact full-length antibodies, but also fragments thereof (such as Fab, Fab', F(ab')₂, Fv), single chain (ScFv), mutants thereof, fusion proteins comprising an antibody portion, and any other modified configuration of the immunoglobulin molecule that comprises an antigen recognition site of the required specificity. An antibody includes an antibody of any class, such as IgG, IgA, or IgM (or sub-class thereof), and the antibody need not be of any particular class. Depending on the antibody amino acid sequence of the constant domain of its heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1 and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known.

"Fc" refers to the "fragment crystallizable" portion of an antibody molecule, the heavy chain constant region of an antibody that corresponds to an antibody class and interacts with a cell surface receptor.

"Hinge" refers to the flexible region of an antibody molecule between the constant and variable regions of the heavy chain.

"Chimeric polypeptide" or "fusion protein" refers to a protein that is created through genetic engineering such that two or more separate proteins are expressed as a single polypeptide through the joining of genes that originally coded for the separate proteins.

"Linker" refers to a short peptide sequence between two polypeptides of a fusion protein.

"Pharmaceutically acceptable" means approved by a regulatory agency of the Federal or a state government or listed in the U.S. Pharmacopoeia or other generally recognized pharmacopoeia for use in animals, and more particularly in humans.

"Pharmaceutically acceptable vehicle" or "pharmaceutically acceptable excipient" refers to a diluent, adjuvant, excipient or carrier with which a protein as described herein is administered.

An "individual" or "subject" refers to a vertebrate, typically a mammal, such as a human.

"Treating" or "treatment" of any disease or disorder refers, in one embodiment, to ameliorating the disease or disorder (*i.e.,* arresting or reducing the development of the disease or at least one of the clinical symptoms thereof). In another embodiment "treating" or "treatment" refers to ameliorating at least one physical parameter, which may not be discernible by the subject. In yet another embodiment, "treating" or "treatment" refers to modulating the disease or disorder, either physically (*e.g.,* stabilization of a discernible symptom), physiologically (*e.g.,* stabilization of a physical parameter), or both.

"Therapeutically effective amount" means the amount of a compound that, *i.e.,* a protein as described herein, that when administered to an individual for treating a disease or condition, is sufficient to effect such treatment for the disease or condition or to reduce severity of or eliminate at least one symptom of the disease or condition. "Therapeutically effective amount" means that amount of the compound that will elicit the biological or medical response of a subject that is being sought by a medical doctor or other clinician. The "therapeutically effective amount" can vary depending on the compound, the disease and its severity, and the age, weight, etc., of the subject to be treated.

"Prophylaxis" means a measure taken for the prevention of a disease or condition or at least one symptom thereof.

"Preventing" or "prevention" refers to a reduction in risk of acquiring a disease or disorder (*i.e.,* causing at least one of the clinical symptoms of the disease not to develop in a subject that may be exposed to or predisposed to the disease but does not yet experience or display symptoms of the disease, or causing the symptom to develop with less severity than in absence of the treatment). "Prevention" or "prophylaxis" may refer to delaying the onset of the disease or disorder.

"Prophylactically effective amount" means the amount of a compound that, *i.e.,* a protein as described herein, that when administered to an individual for prevention of a disease or condition, is sufficient to effect such prevention of the disease or condition or to prevent development of at least one symptom of the disease or condition or effect development of the symptom at a lower level of severity than in the absence of administration of the compound. The "prophylactically effective amount" can vary depending on the compound, the disease and its severity, and the age, weight, etc., of the subject to be treated.

A "biologically active polypeptide" refers to a polypeptide that is capable of a biological activity. For example, in some embodiments, a biologically active peptide may be used for a therapeutic purpose, for example, in a method of treatment of a disease condition.

The term "unit dosage form" refers to physically discrete units suitable as a unitary dosages for an individual to whom administered, each unit containing a predetermined quantity of active material calculated to produce the desired therapeutic or prophylactic effect, in association with a suitable pharmaceutical excipient. A "unit dose" is an amount of a substance sufficient to provide a therapeutically or prophylactically effective level, e.g., plasma level, in the individual for an amount of time.

### Chimeric polypeptides

Chimeric polypeptides are provided. The chimeric polypeptides include the hinge region of IgG1 and the Fc region of IgG4, or variants thereof, in the formula H-Fc. In some embodiments, the polypeptide may include a human IgG1 hinge polypeptide or a variant thereof, for example, comprising or consisting of the amino acid sequence depicted in SEQ ID NO:1, SEQ ID NO:2, or SEQ ID NO:3, or a sequence having at least about 80, 85, 90, 95, 98, or 99% sequence identity thereof. In some embodiments, the polypeptide may include a human IgG4 Fc polypeptide or a variant thereof, for example, comprising or consisting of the amino acid sequence depicted in SEQ ID NO:4, or a sequence having at least about 80, 85, 90, 95, 98, or 99% sequence identity thereof.

In some embodiments, the polypeptide may further include a linker polypeptide, in the formula L-H-Fc. A linker polypeptide provides flexibility between the IgG1 hinge sequence and a biologically active polypeptide, when present. In some embodiments, the linker sequence may be about 5 to about 20, about 5 to about 10, about 10 to about 15, about 5 to about 15, about 10 to about 20, or about 15 to about 20 amino acids in length. Typically, the linker polypeptide includes properties of solubility, flexibility, and low immunogenicity. In one embodiment, the linker sequence comprises or consists of the amino acid sequence depicted in SEQ ID NO:5.

In some embodiments, the polypeptide may further include a biologically active polypeptide, in the formula P-L-H-Fc or P-H-Fc. For example, the biologically active polypeptide may be a polypeptide with therapeutic, prophylactic, or beneficial effect for an individual in need thereof. In various embodiments, the biologically active polypeptide may be a hormone, a cytokine, a growth factor, a co-stimulatory molecule, a hormone receptor, a cytokine receptor, a growth factor receptor, a short peptide, or an antibody fragment (*e.g.,* a Fab fragment, a V_{H} fragment, a V_{L} fragment, or a camelid nanobody). For example, the biologically active polypeptide may be hGH, GLP-1, TNFR, Factor VIII, VEGFR, or TGF-beta1. In one embodiment, the biologically active polypeptide is hGH, *e.g.,* comprising or consisting of the amino acid sequence depicted in SEQ ID NO:6, or a sequence having at least about 80, 85, 90, 95, 98, or 99% sequence identity thereof.

The chimeric polypeptide may be in the form of a dimer. For example, the polypeptides of the dimer may be joined by at least one interchain disulfide bond in the hinge regions. In some embodiments, the dimer includes one or two interchain disulfide bonds in the hinge region.

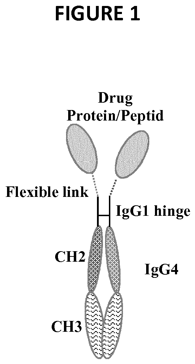

In one embodiment, the chimeric polypeptide is the construct depicted schematically in Figure 1, with the formula P-L-H-Fc, where P is any biologically active polypeptide, L is a flexible linker sequence, H is the hinge of IgG1 (*e.g.,* human IgG1), and Fc is the Fc of IgG4 (*e.g.,* human IgG4).

### Polynucleotides

Polynucleotides are provided that encode chimeric polypeptides as described herein. A polynucleotide may encode a chimeric polypeptide of the formula H-Fc, L-H-Fc, P-L-H-Fc, or P-H-Fc, as described above.

In some embodiments, the polynucleotide comprises a sequence encoding an IgG1 hinge (H) polypeptide or a variant thereof, *e.g.,* a human IgG1 hinge polypeptide or a variant thereof, for example, the amino acid sequence depicted in SEQ ID NO:1, SEQ ID NO:2, or SEQ ID NO:3, or a sequence having at least about 80, 85, 90, 95, 98, or 99% sequence identity thereof.

In some embodiments, the polynucleotide comprises a sequence encoding an IgG4 Fc polypeptide or a variant thereof, *e.g.,* a human IgG4 Fc polypeptide or a variant thereof, for example, the amino acid sequence depicted in SEQ ID NO:4, or a sequence having at least about 80, 85, 90, 95, 98, or 99% sequence identity thereof.

In some embodiments, the polynucleotide comprises a sequence encoding a linker (L) polypeptide. In one embodiment, the linker sequence comprises or consists of the amino acid sequence depicted in SEQ ID NO:5.

In some embodiments, the polynucleotide encodes one or more biologically active polypeptide (P). For example, the polynucleotide may comprise a sequence encoding a hormone, a cytokine, a growth factor, a co-stimulatory molecule, a hormone receptor, a cytokine receptor, a growth factor receptor, a short peptide, or an antibody fragment. For example, the polynucleotide may comprise a sequence encoding hGH, GLP-1, TNFR, Factor VIII, VEGFR, or TGF-beta1. In one embodiment, the polynucleotide comprises a sequence encoding hGH, for example, the amino acid sequence depicted in SEQ ID NO:6, or a sequence having at least about 80, 85, 90, 95, 98, or 99% sequence identity thereof.

In some embodiments, the polynucleotide is operably linked to promoter(s) for expression in a host microorganism. In some embodiments, the polynucleotide is codon optimized for expression in a microorganism in which it will be expressed.

In some embodiments, the polynucleotide is included within a vector, such as an expression vector.

### Host cells

Host cells are provided for production of chimeric polypeptides. The host cell includes an exogenous polynucleotide that encodes a chimeric polypeptide as described herein. Regulatory sequences for expression of the chimeric polypeptide are either provided as endogenous polynucleotide sequences of the host cell or are added as exogenous sequences. In some embodiments, the host cell is transformed with a vector, such as an expression vector, that includes a polynucleotide that encodes the chimeric polypeptide. In other embodiments, the exogenous polynucleotide is integrated into the genome of the host cell, for example, with sequences for its expression.

In some embodiments, the host cell is a bacterial cell, such as an *E. coli* cell.

### Microbial cultures

Methods are provided for producing the protein described herein. The methods include culturing a host cell as described above, under conditions in which the protein is expressed from the exogenous polynucleotide that encodes it. The chimeric polypeptide may be recovered and/or purified from the host cells and/or from the culture medium in which the host cells are cultured. In some embodiments, the chimeric polypeptide is produced in soluble form, e.g., as a soluble dimer, and is purified from the host cells, e.g., from the cytoplasm of the host cells.

Culture media for the production of the chimeric polypeptide include a carbon source, such as a source of fermentable carbohydrate molecules.

The culture medium includes carbon source(s), nitrogen source(s), inorganic substances (*e.g.,* inorganic salts), and any other substances required for the growth of the microorganism (*e.g.,* vitamins, amino acids, etc.).

The carbon source may include sugars, such as glucose, sucrose, lactose, fructose, trehalose, mannose, mannitol, and/or maltose; organic acids, such as acetic acid, lactic acid, fumaric acid, citric acid, propionic acid, malic acid, pyruvic acid, malonic acid, succinic acid, and/or ascorbic acid; alcohols, such as methanol, ethanol, propanol, butanol, pentanol, hexanol, isobutanol, and/or glycerol; oil or fat, such as soybean oil, rice bran oil, olive oil, corn oil, sesame oil, and/or linseed oil, and the like. The amount of the carbon source added varies according to the kind of the carbon source, for example, about 1 to about 100 g, or about 2 to about 50 g per liter of medium.

As known in the art, in addition to an appropriate carbon source, fermentation media must contain suitable nitrogen source(s), mineral salts, cofactors, buffers, and other components suitable for the growth of the cultures and production of the desired bioproduct, *i.e.,* the chimeric polypeptide. In some embodiments, salts and/or vitamin B12 or precursors thereof are included in the fermentation media.

The nitrogen source may be any suitable nitrogen source, including but not limited to, ammonium salts, yeast extract, corn steep liquor (CSL), and/or other protein sources including, but not limited to, denatured proteins recovered from distillation of fermentation broth or extracts derived from the residual separated microbial cell mass recovered after fermentation. The nitrogen source may include potassium nitrate, ammonium nitrate, ammonium chloride, ammonium sulfate, ammonium phosphate, ammonia, urea, and the like, alone or in combination. Amount of the nitrogen source added varies according to the kind of the nitrogen source, for example, about 0.1 to about 30 g, or about 1 to about 10 g per liter of medium.

Inorganic salts may include potassium dihydrogen phosphate, dipotassium hydrogen phosphate, disodium hydrogen phosphate, sodium dihydrogen phosphate, magnesium sulfate, magnesium chloride, ferric sulfate, ferrous sulfate, ferric chloride, ferrous chloride, manganese sulfate, manganese chloride, zinc sulfate, zinc chloride, cupric sulfate, calcium chloride, calcium carbonate, sodium carbonate, sodium sulfate, and the like, alone or in combination. Amount of inorganic salt varies according to the kind of the inorganic salt, for example, about 0.00001 to about 10 g per liter of medium.

Phosphorus may be present in the medium in the form of phosphate salts, such as sodium, potassium, and/or ammonium phosphates. Sulfur may be present in the medium in the form of sulfate salts, such as sodium and/or or ammonium sulfates. Additional salts include, but are not limited to, magnesium sulfate, manganese sulfate, iron sulfate, magnesium chloride, calcium chloride, manganese chloride, ferric chloride, ferrous chloride, zinc chloride, cupric chloride, cobalt chloride, and/or sodium molybdate. The growth medium may also contain vitamins such as thiamine hydrochloride, biotin, and/or para-aminobenzoic acid (PABA). The growth medium may also contain one or more buffering agent(s) (*e.g.,* MES), one or more reducing agent(s) (*e.g.,* cysteine HCI), and/or sodium lactate, which may serve as a carbon source and pH buffer.

Special required substances, for example, vitamins, nucleic acids, yeast extract, peptone, meat extract, malt extract, corn steep liquor, soybean meal, dried yeast etc., may be included alone or in combination. Amount of the special required substance used varies according to the kind of the substance, for example, about 0.2 g to about 200 g, or about 3 to about 10 g per liter of medium.

In some embodiments, the culture conditions include growth of the host cell at an ambient temperature, such as about 20° C to about 25° C. In an embodiment, the host cell is a bacterial cell, the temperature of the growth medium is about 20° C to about 25° C, and the chimeric polypeptide is produced as a soluble dimer in the cytoplasm of the host cell, *e.g.,* not sequestered in inclusion bodies.

In some embodiments, the host cell is a bacterial cell, such as an *E. coli* cell. In an embodiment, the host cell is an *E. coli* cell, the temperature of the growth medium is about 20° C to about 25° C, and the chimeric polypeptide is produced as a soluble dimer in the cytoplasm of the host cell *e.g.,* not sequestered in inclusion bodies.

In certain embodiments, the culture conditions may further include mild conditions for induction of beta-galactosidase activity, such as, for example, a low concentration (*e.g.,* about 0.01% (w/w) to about 0.8% (w/w)) of an inducing reagent such as isopropyl beta-D-1-thiogalactopyranoside (IPTG) or lactose. In some embodiments, the concentration of the inducing reagent may be any of about 0.01% (w/w) to about 0.05% (w/w), about 0.01% (w/w) to about 0.1% (w.w), about 0.05% (w/w) to about 0.1% (w/w), about 0.1% (w/w) to about 0.5% (w/w), about 0.1% (w/w) to about 0.8% (w/w), or about 0.5% (w/w) to about 0.8% (w/w).

In some embodiments, the chimeric polypeptide is produced, at a titer of about 1 g/l to about 3 g/l. In some embodiments, the chimeric polypeptide is produced at a yield of about 30% to about 50%. For example, in certain embodiments, the chimeric polypeptide is produced in soluble form, such as a soluble dimer, *e.g.,* in a bacterial culture, *e.g.,* an *E. coli* culture, *e.g.,* at ambient temperature, at a titer of about 1 g/l to about 3 g/l and a yield of about 30% to about 50%.

Chimeric polypeptides produced as described herein may be purified from the host cells or from the culture medium. Nonlimiting methods for purification include extraction and/or chromatographic purification. For example, one or more chromatographic purification steps may be deployed, including but not limited to, affinity chromatography, ion-exchange chromatography, hydrophobic chromatography, and size-exclusion chromatography.

### Compositions

Compositions are provided that include a chimeric polypeptide as described herein. In some embodiments, the composition includes the chimeric polypeptide in combination with one or more other substance(s) that are associated with the source of the polypeptide, for example, a cellular extract, *e.g.,* from a production host cell or a culture medium.

In some embodiments, the composition is a pharmaceutical composition, and includes at least one pharmaceutically acceptable excipient. In one embodiment, composition includes a parenteral carrier. In another embodiment, the composition includes an oral carrier. In yet another embodiment, the composition includes an intravenous (iv) carrier.

In some embodiments, the composition is formulated for delivery to a desired site of action within an individual to whom it is administered.

When employed as pharmaceuticals, *i.e.,* for treatment or prophylaxis of a disease or condition, the compositions described herein are typically administered in the form of a pharmaceutical composition. Such compositions can be prepared in a manner well known in the pharmaceutical art and include at least one active compound, *i.e.,* a biologically active polypeptide as described herein.

Generally, the compositions are administered in a pharmaceutically effective amount, *i.e.,* a therapeutically or prophylactically effective amount. The amount of the active agent, *i.e.,* a biologically active polypeptide as described herein, actually administered will typically be determined by a physician, in the light of the relevant circumstances, including the condition to be treated, the chosen route of administration, the activity of the protein administered, the age, weight, and response of the individual patient, the severity of the patient's symptoms, and the like.

The pharmaceutical compositions can be administered by a variety of routes including oral, rectal, transdermal, subcutaneous, intravenous, intramuscular, and intranasal. Depending on the intended route of delivery, the pharmaceutical compositions are preferably formulated as either injectable or oral compositions or as salves, as lotions or as patches for transdermal administration.

The compositions for oral administration can take the form of bulk liquid solutions or suspensions, or bulk powders. More commonly, however, the compositions are presented in unit dosage forms to facilitate accurate dosing. Typical unit dosage forms include prefilled, premeasured ampules or syringes of the liquid compositions or pills, tablets, capsules or the like in the case of solid compositions. In some embodiments of such compositions, active agent, *i.e.,* a chimeric polypeptide with a biologically active polypeptide as described herein, may be a minor component (about 0.1% to about 50% by weight, or about 1% to about 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable carriers known in the art. As described above, the active agent, in such compositions, i.e., a chimeric polypeptide with a biologically active polypeptide as described herein, is typically a minor component, often being about 0.05% to 10% by weight, with the remainder being the injectable carrier and the like.

The above-described components for orally administrable, or injectable administrable compositions are merely representative. Other materials as well as processing techniques and the like are set forth in Part 8 of Remington's The Science and Practice of Pharmacy, 21st edition, 2005, Publisher: Lippincott Williams & Wilkins, which is incorporated herein by reference.

The chimeric polypeptide described herein can also be administered in sustained release forms or from sustained release drug delivery systems. A description of representative sustained release materials can be found in Remington's Pharmaceutical Sciences.

### Methods of treatment

Methods are provided for treating, preventing, or ameliorating at least one symptom of a disease or condition, including administering a therapeutically or prophylactically effective amount of biologically active polypeptide in the form of a chimeric polypeptide, *e.g.,* a chimeric polypeptide dimer, as described herein (*e.g.,* a pharmaceutical composition that includes a chimeric polypeptide containing a biologically active polypeptide, *e.g.,* a dimer of chimeric polypeptides each containing a biologically active polypeptide) to an individual in need thereof, *i.e.,* an individual suffering from or at risk of developing the disease or condition or at least one symptom of the disease or condition. Administration of the biologically active polypeptide prevents, reduces the severity or, or eliminates at least one symptom of the disease or condition in the individual. The individual may be a mammal, such as, but not limited to, a human.

Diseases or conditions treatable with the compositions described herein include, but are not limited to, GHD, diabetes (*e.g.,* Type II diabetes), arthritis (*e.g.,* rheumatoid arthritis), and hemophilia, for example, with biologically active polypeptides hGH, glucagon-like peptide 1 (GLP - 1), tumor necrosis factor receptor (TNFR), and Factor VIII, respectively.

In one embodiment, the disease or condition is GHD, and the biologically active polypeptide is hGH.

In various embodiments, the chimeric polypeptide may be administered orally, or parenterally (*e.g.*, subcutaneously (s.c.), intramuscularly (i.m.), intravenously (i.v.), intraperitoneally (i.p.), or intrathecally (i.t.) In some embodiments of the methods herein, the pharmaceutical composition is formulated for subcutaneous administration and is administered subcutaneously.

In some embodiments, the chimeric polypeptide has a longer circulating half life in the individual than the biologically active polypeptide administered alone. For example, the circulating half life may be at least about 5-fold, 6-fold, 7-fold, 8-fold, 9-fold, or 10-fold higher, or about 5-fold to about 12-fold fold higher.

For example, the chimeric polypeptide may be administered, e.g., subcutaneously administered, weekly, bi-weekly, twice monthly), or monthly.

In some embodiments, the chimeric polypeptide is administered weekly at a dosage of about 0.8 mg/kg to about 1.2 mg/kg, or is administered twice monthly at a dosage of about 1.6 mg/kg or about 2.4 mg/kg. For example, in one embodiment, the chimeric polypeptide includes hGH and is administered subcutaneously to treat GHD weekly at a dosage of about 0.8 mg/kg to about 1.2 mg/kg, or is administered twice monthly at a dosage of about 1.6 mg/kg or about 2.4 mg/kg. The dosing schedule and actual dosage administered may vary depending on such factors as the nature and severity of the disease or condition, the age, weight, and general health of the patient and the tolerance of a particular patient to the biologically active polypeptide, but will be ascertainable to health professionals. The amount of chimeric polypeptide administered may be any of the dosages disclosed herein. The dose is generally chosen such that the biologically active polypeptide will remain at a therapeutically or prophylactically effective plasma level for an extended period of time, often at least 5 days, more often about one week, two weeks, or longer.

In some embodiments of the methods herein, a second therapeutic or prophylactic agent is administered in conjunction with the chimeric polypeptide described herein, either simultaneously or sequentially. In some embodiments, the protein and the second agent act synergistically for treatment or prevention of the disease or condition or symptom(s). In other embodiments, the protein and the second agent act additively for treatment or prevention of the disease or condition or symptom(s).

### Kits

Kits are provided for preparation and/or use of chimeric polypeptides as described herein. For example, a kit may include a polynucleotide, for example, in an expression vector, optionally in a host cell, for production of a chimeric polypeptide as described herein, or a kit may include a chimeric polypeptide for use in a therapeutic method as described herein.

In some embodiments, the kit includes an expression vector that includes a polynucleotide encoding a chimeric polypeptide as described herein, such as a IgG1 hinge - IgG4 Fc fusion polypeptide, or a linker - IgG1 hinge - IgG4 Fc fusion, or a biologically active polypeptide - optional linker - IgG1 hinge - IgG4 Fc fusion. In some embodiments, the expression vector may be provided in a host cell, such as a bacterial cell, e.g., an *E. coli* cell. Instructions for expression and production of the chimeric polypeptide, for example, in a bacterial culture, may be provided. Optionally, culture media, or components of culture media, such as nutrients, buffers, etc. may be included in the kit.

In some embodiments, the kit includes a composition, *e.g.,* a chimeric polypeptide or a pharmaceutical composition thereof, for example including one or more unit dose of a chimeric polypeptide that includes a biologically active polypeptide. Optionally, instructions for use and/or administration, e.g., subcutaneous administration, of the composition, in a method described herein, are provided.

Instructions for a kit as described herein may be provided in printed form or in the form of an electronic medium such as a CD or DVD, or in the form of a website address where such instructions may be obtained or a mobile application.

A kit may be provided in suitable packaging. As used herein, "packaging" refers to a solid matrix or material customarily used in a system and capable of holding within fixed limits a composition suitable for use in a method as described herein. Such materials include glass and plastic (e.g., polyethylene, polypropylene, and polycarbonate) bottles, vials, paper, plastic, and plastic-foil laminated envelopes and the like. If e-beam sterilization techniques are employed, the packaging should have sufficiently low density to permit sterilization of the contents.

The following examples are intended to illustrate, but not limit, the invention.

### EXAMPLES

### Example 1

### Preparation of Expression Vector for MC2-B

A codon-optimized polynucleotide sequence encoding the hGH fusion polypeptide "MC2-B" was synthesized including hGH, linker, hinge region of IgG1, and Fc region of IgG4, as shown schematically in **Fig. 1****.**

The synthetic gene was cloned into a vector pET26b (Novagen) according to standard DNA manipulation methods. Accuracy of cloning was verified using DNA sequencing.

Then, the recombinant plasmid was transformed into chemically competent *E. coli* BL21 (DE3) cells and the colonies were selected on a LB plate supplemented with 50 µg/ml Kanamycin. The transformant was designated BL21/MC-2B (MC040617).

### Example 2

### Expression of MC2-B

First, each transformant was grown in 100 ml of LB medium with agitation overnight, and inoculated in a fermenter for large-scale culture. The fermenter was maintained at 25 °C or 37 °C. To compensate for the insufficient nutrients for bacterial growth during fermentation, the cultures were supplemented with glucose and yeast extract according to the fermentation states of the bacteria. When the cultures reached an OD600 value of 3, the fermenter was cooled down to 20 °C, and an inducer, lactose, was added to the cultures to induce protein expression. The cultures were further cultured for 12 to 18 hrs to increase the OD value at 600 nm to 30 to 40.

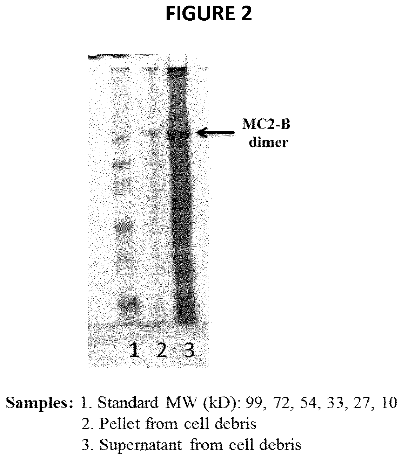

The expression level of soluble MC2-B in the *E. coli* transformant was examined as follows. Cells were disrupted in disruption buffer 20 mM TrisHCI, pH8.5; 1 mM EDTA by a sonicator. The cell lysate thus obtained was centrifuged to separate water-soluble substances from water-insoluble substances. Portions of cell disrupted supernatants and pellets were mixed with equal volumes of 2× protein sample buffer and electrophoresed on a 15% SDS-PAGE gel. As a result, as shown in **Fig. 2****,** MC2-B was observed to be overexpressed mostly in supernatants, indicating that MC2-B was expressed in soluble form in *E. coli.*

### Example 3

### Purification of MC2-B

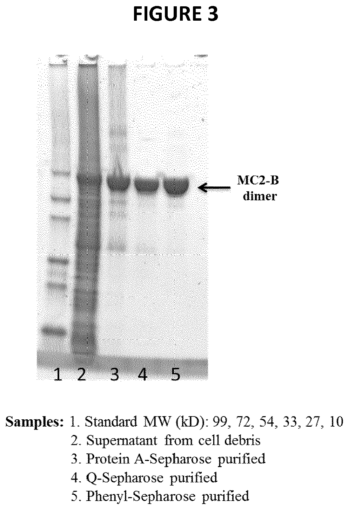

The washed cell pellets were suspended in disruption buffer 20 mM TrisHCI, pH8.5; 1 mM EDTA and disrupted by a high-pressure homogenizer. Supernatants collected by centrifugation were incubated overnight at 4° C, and then purified through column chromatography. After 5 ml of a protein-A affinity column (Pharmacia) was equilibrated with phosphate buffered saline (PBS), the cell lysates were loaded onto the column at a flow rate of 5 ml/min. Unbound proteins were washed out with PBS, and bound proteins were eluted with 100 mM glycine (pH 3.0). The collected fractions were neutralized with 1 M TrisHCI, pH8.5, and diluted 1:2 with 20 mM Tris buffer (pH 8.5). Then, the Protein A column purified MC2-B fractions were loaded onto 5 ml column of a HiTrap Q HP (GE Healthcare Life Sciences). The column was eluted with a linear gradient (0.1-0.3 M NaCI) in 20 mM Tris buffer, pH8.5, pure MC2-B fractions then were collected. As a result, shown in **Fig. 3****,** MC2-B was isolated to high purity determined by SDS-PAGE analysis.

### Example 4

### Human growth hormone receptor binding assay of purified MC2-B

To evaluate the binding affinity of the MC2-B fusion protein, a receptor binding enzyme-linked immunosorption assay (ELISA) was performed. The wells of a 96-well plate were coated with 2 µg/mL of recombinant human growth hormone receptor Fc chimera (hGHR-Fc; R&D Systems), and then thoroughly blocked with a solution of 3% bovine serum albumin (BSA) in phosphate-buffered saline (PBS). Purified hGH or MC2-B was serially diluted in Binding Buffer (PBS containing 0.05% Tween-20 and 1% BSA), and incubated on the hGHR-Fc coated wells for at least 1 h at room temperature. Unbound hGH or MC2-B was removed by washing with PBS containing 0.05% Tween-20. A biotinylated polyclonal anti-hGH antibody was used to detect the bound hGH or MC2-B. Streptavidin-horseradish peroxidase (HRP) was then added, excess removed by washing, and bound amount measured by colorimetric change after addition of a 3,3',5,5'-Tetramethylbenzidine (TMB) peroxidase substrate.

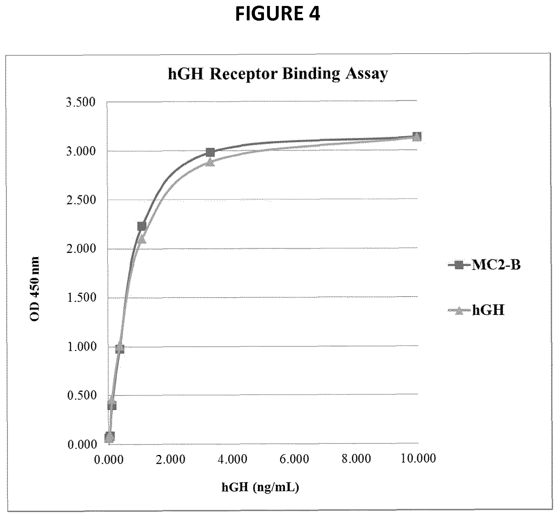

The results are shown in **Fig. 4****.**

### Example 5

### In vitro bioactivity of purified MC2-B

The procedure described in this example involves use of a murine pro-B cell line (BaF/803) that has been stably transfected using hGHR cDNA and pNeo plasmid.18. This cell line, designated BaF/hGHR/B2B2, expresses approximately 3000 surface hGH receptors per cell, and is hGH responsive (as measured by the stimulation of cellular division). Growth and maintenance of this cell line require the inclusion of either rhGH or interleukin 3 into the media during passaging. The mitogenic response to rhGH is quantified by alamar Blue® to detect the proliferation of the BaF/hGHR/B2B2 cells. The effective concentration that results in 50% of maximum growth (EC50) over 2 days is determined from a minimum of eight different concentrations of the test sample or the positive control, rhGH, in triplicate.

### Example 6

### In Vivo Half-life of purified MC2-B

Male Sprague-Dawley rats (approximately 300 g) were divided into three groups (5 per group): one daily hGH group and two MC2-B groups. The hGH group received a single subcutaneous (sc) injection of hGH at a dose of 1 mg/kg in PBS. The MC2-B groups received a single sc injection of MC2-B at doses of 0.5 mg/kg and 1 mg/kg in PBS.

In addition to pre-treatment samples, blood samples of 0.25 ml were collected in EDTA coated micro tubes. Time points were collected at 0.5 h (hour), 1 h, 2 h, 4 h, 8 h, 12 h, 1 d (day), 2 d, 3 d, 4 d, 5 d, 6 d, 7 d, 9 d, 11 d, and 14 d after injection. For the hGH group, blood samples were collected only at 0.5, 1, 2 and 4 h after injection. Samples were stored on ice for up to 1 h prior to centrifugation and plasma harvest. Plasma samples were stored at -20° C prior to analysis.

Concentration of hGH in plasma samples was determined by using a commercial sandwich ELISA kit for detection of hGH (R&D Systems). This kit detects hGH as well as MC2-B by means of an antibody sandwich ELISA format. The concentration of hGH was derived from a standard curve using materials supplied in the kit. The concentration of MC2-B was derived from a standard curve using MC2-B solutions ranging from 0.02 to 4 ng/ml.

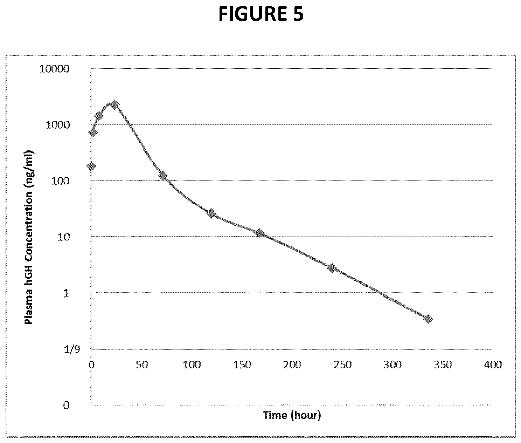

The results are shown in **Fig. 5****.**

### Example 7

### In vivo bioactivity of purified MC2-B; Hypophysectomized (Hpx) Rat Studies

Increase in body weight and the width of the tibial growth plate were utilized as indices of the pharmacological efficacy of hGH. Sprague-Dawley male rats weighing 70-100 g (six per group) had their pituitaries surgically removed at age 3-4 weeks (Hpx rats). Prior to initiation of the efficacy study, the Hpx rats were acclimatized for 1 week and monitored for body weight changes. Hpx rats that demonstrated >10% increase in body weight relative to their initial weight were considered to be incompletely Hypophysectomized and were excluded from the study.

Rats were then randomly divided into four treatment groups (6 rats per group): a control group (no treatment), a daily hGH group, and two MC2-B groups. Rats in the daily hGH group received daily sc injections of hGH at a dose of 15 µg/animal for 14 days. The MC2-B groups received sc injection of MC2-B at doses of 240 µg/animal or 480 µg/animal in PBS once per week for 2 weeks. All injections were given in a volume of 0.2 ml, and the control group received no treatment.

Body weights were measured at 9:00-10:00 am each day prior to injection. After 14 days of treatment, the animals were euthanized.

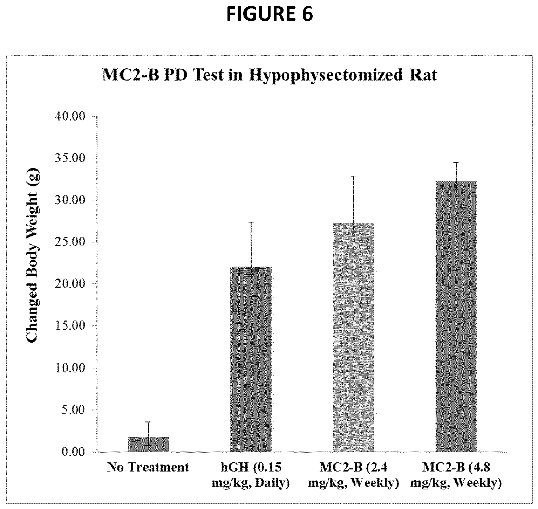

The results are shown in **Fig. 6****.**

### Polypeptide Sequences

SEQ ID NO:1
   Native hinge sequence
   Asp Lys Thr His Thr Cvs Pro Pro Cvs Pro
SEQ ID NO:2
   Hinge mutation 1
   Asp Lys Thr His Thr Ser Pro Pro Cys Pro
SEQ ID NO:3
   Hinge mutation 2
   Asp Lys Thr His Thr Cys Pro Pro Ser Pro
SEQ ID NO:4
   Fc 
Ala Pro Glu Phe Leu Gly Gly Pro Ser Val Phe Leu Phe Pro Pro Lys Pro Lys Asp Thr Leu Met Ile Ser
Arg Thr Pro Glu Val Thr Cys Val Val Val Asp Val Ser Gln Glu Asp Pro Glu Val Gln Phe Asn Trp Tyr
Val Asp Gly Val Glu Val His Asn Ala Lys Thr Lys Pro Arg Glu Glu Gln Phe Asn Ser Thr Tyr Arg Val
Val Ser Val Leu Thr Val Leu His Gln Asp Trp Leu Asn Gly Lys Glu Tyr Lys Cys Lys Val Ser Asn Lys
Gly Leu Pro Ser Ser Ile Glu Lys Thr Ile Ser Lys Ala Lys Gly Gln Pro Arg Glu Pro Gln Val Tyr Thr Leu
Pro Pro Ser Gln Glu Glu Met Thr Lys Asn Gln Val Ser Leu Thr Cys Leu Val Lys Gly Phe Tyr Pro Ser
Asp Ile Ala Val Glu Trp Glu Ser Asn Gly Gln Pro Glu Asn Asn Tyr Lys Thr Thr Pro Pro Val Leu Asp
Ser Asp Gly Ser Phe Phe Leu Tyr Ser Arg Leu Thr Val Asp Lys Ser Arg Trp Gln Glu Gly Asn Val Phe
Ser Cys Ser Val Met His Glu Ala Leu His Asn His Tyr Thr Gln Lys Ser Leu Ser Leu Ser Pro Gly Lys
SEQ ID NO:5
   Linker sequence
   Gly Gly Gly Gly Ser
SEQ ID NO:6
   hGH

Met Phe Pro Thr Ile Pro Leu Ser Arg Leu Phe Asp Asn Ala Met Leu Arg Ala His Arg Leu His Gln Leu
Ala Phe Asp Thr Tyr Gln Glu Phe Glu Glu Ala Tyr Ile Pro Lys Glu Gln Lys Tyr Ser Phe Leu Gln Asn
Pro Gln Thr Ser Leu Cys Phe Ser Glu Ser Ile Pro Thr Pro Ser Asn Arg Glu Glu Thr Gln Gln Lys Ser
Asn Leu Glu Leu Leu Arg Ile Ser Leu Leu Leu Ile Gln Ser Trp Leu Glu Pro Val Gln Phe Leu Arg Ser
Val Phe Ala Asn Ser Leu Val Tyr Gly Ala Ser Asp Ser Asn Val Tyr Asp Leu Leu Lys Asp Leu Glu Glu
Gly Ile Gln Thr Leu Met Gly Arg Leu Glu Asp Gly Ser Pro Arg Thr Gly Gln Ile Phe Lys Gln Thr Tyr
Ser Lys Phe Asp Thr Asn Ser His Asn Asp Asp Ala Leu Leu Lys Asn Tyr Gly Leu Leu Tyr Cys Phe
Arg Lys Asp Met Asp Lys Val Glu Thr Phe Leu Arg Ile Val Gln Cys Arg Ser Val Glu Gly Ser Cys Gly
Phe

Although the foregoing invention has been described in some detail by way of illustration and examples for purposes of clarity of understanding, it will be apparent to those skilled in the art that certain changes and modifications may be practiced without departing from the spirit and scope of the invention. Therefore, the description should not be construed as limiting the scope of the invention.

All publications, patents, and patent applications cited herein are hereby incorporated by reference in their entireties for all purposes and to the same extent as if each individual publication, patent, or patent application were specifically and individually indicated to be so incorporated by reference.

The present invention will now be described with reference to the following numbered embodiments:
Embodiment 1 is a chimeric polypeptide, comprising the formula: H - Fc, wherein H is the hinge region of IgG1, and Fc is the Fc region of IgG4.
Embodiment 2 is a chimeric polypeptide according to Embodiment 1, wherein H and Fc are human polypeptide sequences.
Embodiment 3 is a chimeric polypeptide according to Embodiment 2, wherein H comprises the amino acid sequence depicted in SEQ ID NO:1, or a sequence having at least about 80% sequence identity thereof.
Embodiment 4 is a chimeric polypeptide according to Embodiment 2, wherein H comprises the amino acid sequence depicted in SEQ ID NO:2.
Embodiment 5 is a chimeric polypeptide according to Embodiment 2, wherein H comprises the amino acid sequence depicted in SEQ ID NO:3.
Embodiment 6 is a chimeric polypeptide according to Embodiment 2, wherein Fc comprises the amino acid sequence depicted in SEQ ID NO:4, or a sequence having at least about 80% sequence identity thereof.
Embodiment 7 is a chimeric polypeptide according to Embodiment 1, further comprising the formula: L-H-Fc, wherein L is a peptide linker.
Embodiment 8 is a chimeric polypeptide according to Embodiment 7, wherein L comprises about 5 to about 10 amino acids.
Embodiment 9 is a chimeric polypeptide according to Embodiment 8, wherein L comprises the amino acid sequence depicted in SEQ ID NO:5.
Embodiment 10 is a chimeric polypeptide according to Embodiment 7, further comprising the formula: P-L-H-Fc, wherein P is a biologically active polypeptide.
Embodiment 11 is a chimeric polypeptide according to Embodiment 10, wherein the biologically active polypeptide comprises a hormone, a cytokine, a growth factor, a co-stimulatory molecule, a hormone receptor, a cytokine receptor, a growth factor receptor, a short peptide, or an antibody fragment.
Embodiment 12 is a chimeric polypeptide according to Embodiment 11, wherein the biologically active polypeptide comprises an antibody fragment selected from a Fab fragment, a V_{H} fragment, a V_{L} fragment, or a camelid nanobody.
Embodiment 13 is a chimeric polypeptide according to Embodiment 10, wherein the biologically active polypeptide comprises human growth hormone (hGH).
Embodiment 14 is a chimeric polypeptide according to Embodiment 13, wherein the hGH comprises the amino acid sequence depicted in SEQ ID NO:6, or a sequence having at least about 80% sequence identity thereof.
Embodiment 15 is a chimeric polypeptide according to Embodiment 10, wherein the biologically active polypeptide is selected from GLP-1, TNFR, Factor VIII, VEGFR, and TGF-beta1.
Embodiment 16 is a dimerized chimeric polypeptide, comprising a dimer of identical chimeric polypeptides according to any preceding Embodiment, wherein the dimer comprises at least one interchain disulfide bond in the hinge region.
Embodiment 17 is a dimerized chimeric polypeptide according to Embodiment 16, wherein the dimer comprises one or two interchain disulfide bonds in the hinge region.
Embodiment 18 is a dimerized chimeric polypeptide, comprising a dimer of identical chimeric polypeptides that comprise a biologically active polypeptide, according to any of Embodiments 10 to 15, wherein the dimer comprises at least one interchain disulfide bond in the hinge region.
Embodiment 19 is a polynucleotide that encodes a chimeric polypeptide according to any of Embodiments 1 to 15.
Embodiment 20 is an expression vector comprising a polynucleotide according to Embodiment 19.
Embodiment 21 is a host cell comprising an expression vector according to Embodiment 20.
Embodiment 22 is a host cell according to Embodiment 21, wherein the host cell is a bacterial cell.
Embodiment 23 is a host cell according to Embodiment 22, wherein the host cell is an E. *coli* cell.
Embodiment 24 is a method of producing a chimeric polypeptide, comprising culturing a host cell according to Embodiment 21 under conditions suitable for expression of the chimeric polypeptide.
Embodiment 25 is a method according to Embodiment 24, wherein the host cell is a bacterial cell.
Embodiment 26 is a method according to Embodiment 24, wherein the chimeric polypeptide is produced in a soluble form in the cytoplasm of the host cell.
Embodiment 27 is a method according to Embodiment 26, wherein the chimeric polypeptide is produced as a soluble dimer.
Embodiment 28 is a method according to Embodiment 27, wherein the host cell is an E. *coli* cell.
Embodiment 29 is a method according to Embodiment 28, wherein the culture conditions comprise culturing the host cell at a temperature of about 20°C to about 25°C.
Embodiment 30 is a method according to Embodiment 29, wherein the culture conditions further comprise mild conditions for induction of beta-galactosidase activity.
Embodiment 31 is a method according to Embodiment 30, wherein the mild conditions for induction of beta-galactosidase activity comprise a low concentration of an inducing reagent selected from isopropyl beta-D-1-thiogalactopyranoside (IPTG) or lactose.
Embodiment 32 is a method according to Embodiment 29, wherein the chimeric polypeptide is produced at a titer of about 1 g/liter to about 3 g/liter and a yield of about 30% to about 50%.
Embodiment 33 is a method according to Embodiment 26, wherein the chimeric polypeptide is purified from the host cells by a method comprising extraction and/or chromatographic purification.
Embodiment 34 is a method according to Embodiment 33, wherein purification of the chimeric polypeptide comprises one or more chromatographic purification steps selected from affinity chromatography, ion-exchange chromatography, hydrophobic chromatography, size-exclusion chromatography, and combinations thereof.
Embodiment 35 is a pharmaceutical composition, comprising a chimeric polypeptide according to Embodiment 18, and a pharmaceutically acceptable carrier.
Embodiment 36 is a dosage form comprising a therapeutically effective dose of a pharmaceutical composition according to Embodiment 35.
Embodiment 37 is a method of treatment of a condition, comprising administering a therapeutically effective amount of a pharmaceutical composition according to Embodiment 35 to an individual in need thereof, wherein said biologically active polypeptide treats said condition.
Embodiment 38 is a method according to Embodiment 37, wherein the individual is a mammal.
Embodiment 39 is a method according to Embodiment 38, wherein the mammal is a human.
Embodiment 40 is a method according to Embodiment 39, wherein said condition comprises hGH deficiency, and wherein said biologically active polypeptide comprises hGH.
Embodiment 41 is a method according to Embodiment 37, wherein said biologically active polypeptide comprises a longer circulating half life in the individual than the biologically active polypeptide alone.
Embodiment 42 is a method according to Embodiment 41, wherein the circulating half life of the biologically active polypeptide in the individual is increased by about 6% to about 10%, in comparison to the circulating half life of the biologically active polypeptide alone.
Embodiment 43 is a method according to Embodiment 37, wherein the said administration comprises subcutaneous injection.
Embodiment 44 is a method according to Embodiment 37, wherein the therapeutically effective amount comprises a weekly dosage of about 0.8 mg/kg to about 1.2 mg/kg or a twice monthly dosage of about 1.6 mg/kg to about 2.4 mg/kg.
Embodiment 45 is a kit comprising an expression vector according to Embodiment 20.
Embodiment 46 is a kit comprising a host cell according to Embodiment 21.

## Claims

1. A chimeric polypeptide, comprising the formula:
H - Fc,
wherein H is the hinge region of IgG1, and Fc is the Fc region of IgG4.

2. A chimeric polypeptide according to claim 1, wherein H and Fc are human polypeptide sequences.

3. A chimeric polypeptide according to claim 2, wherein H comprises the amino acid sequence depicted in SEQ ID NO:1, or a sequence having at least about 80% sequence identity thereof; or wherein H comprises the amino acid sequence depicted in SEQ ID NO:2; or wherein H comprises the amino acid sequence depicted in SEQ ID NO:3; or wherein Fc comprises the amino acid sequence depicted in SEQ ID NO:4, or a sequence having at least about 80% sequence identity thereof.

4. A chimeric polypeptide according to claim 1, further comprising the formula:
L-H-Fc,
wherein L is a peptide linker; optionally wherein L comprises about 5 to about 10 amino acids; further optionally wherein L comprises the amino acid sequence depicted in SEQ ID NO:5.

5. A chimeric polypeptide according to claim 4, further comprising the formula:
P-L-H-Fc,
wherein P is a biologically active polypeptide; optionally wherein the biologically active polypeptide comprises a hormone, a cytokine, a growth factor, a co-stimulatory molecule, a hormone receptor, a cytokine receptor, a growth factor receptor, a short peptide, or an antibody fragment; optionally wherein the antibody fragment is selected from a Fab fragment, a V_{H} fragment, a V_{L} fragment, or a camelid nanobody.

6. A chimeric polypeptide according to claim 5, wherein the biologically active polypeptide is selected from human growth hormone (hGH), GLP-1, TNFR, Factor VIII, VEGFR, and TGF-beta1; optionally wherein the hGH comprises the amino acid sequence depicted in SEQ ID NO:6, or a sequence having at least about 80% sequence identity thereof.

7. A dimerized chimeric polypeptide, comprising a dimer of identical chimeric polypeptides according to any preceding claim, wherein the dimer comprises at least one interchain disulfide bond in the hinge region; optionally wherein the dimer comprises one or two interchain disulfide bonds in the hinge region.

8. A polynucleotide that encodes a chimeric polypeptide according to any of claims 1 to 6.

9. An expression vector comprising a polynucleotide according to claim 8.

10. A host cell comprising an expression vector according to claim 9; optionally wherein the host cell is a bacterial cell; further optionally wherein the host cell is an *E. coli* cell.

11. A method of producing a chimeric polypeptide, comprising culturing a host cell according to claim 10 under conditions suitable for expression of the chimeric polypeptide; optionally wherein the host cell is a bacterial cell; further optionally wherein the chimeric polypeptide is produced in a soluble form in the cytoplasm of the host cell; optionally wherein the chimeric polypeptide is produced as a soluble dimer; optionally wherein the host cell is an *E. coli* cell; further optionally wherein the culture conditions comprise culturing the host cell at a temperature of about 20°C to about 25°C; further optionally wherein the culture conditions further comprise mild conditions for induction of beta-galactosidase activity; further optionally wherein the mild conditions for induction of beta-galactosidase activity comprise a low concentration of an inducing reagent selected from isopropyl beta-D-1-thiogalactopyranoside (IPTG) or lactose.

12. A method according to claim 11, wherein the chimeric polypeptide is purified from the host cells by a method comprising extraction and/or chromatographic purification; optionally wherein purification of the chimeric polypeptide comprises one or more chromatographic purification steps selected from affinity chromatography, ion-exchange chromatography, hydrophobic chromatography, size-exclusion chromatography, and combinations thereof.

13. A pharmaceutical composition, comprising a chimeric polypeptide according to claim 7, and a pharmaceutically acceptable carrier.

14. A therapeutically effective amount of a pharmaceutical composition according to claim 13for use as a medicament, wherein the use comprises administration, optionally subcutaneous injection, to an individual in need thereof; optionally wherein the therapeutically effective amount comprises a weekly dosage of about 0.8 mg/kg to about 1.2 mg/kg or a twice monthly dosage of about 1.6 mg/kg to about 2.4 mg/kg; optionally wherein said biologically active polypeptide comprises a longer circulating half life in the individual than the biologically active polypeptide alone; optionally wherein the circulating half life of the biologically active polypeptide in the individual is increased by about 6% to about 10%, in comparison to the circulating half life of the biologically active polypeptide alone.

15. A therapeutically effective amount of a pharmaceutical composition according to claim 13 for use in treating hGH deficiency, and wherein said biologically active polypeptide of said chimeric polypeptide comprises hGH.
